# FASCICULE DE BREVET EUROPEEN

(11) **EP 1 121 461 B1**
(45) Date de publication et mention de la délivrance du brevet: **26.12.2007**
(21) Numéro de dépôt: 99947589.0
(22) Date de dépôt: 15.10.1999
(51) Int. Cl.: C12Q 1/68, G01N 33/58

(54) **SONDES FLUORESCENTES DE PEINTURE CHROMOSOMIQUE**
FLUORESZENZSONDEN ZUM ANFÄRBEN VON CHROMOSOMEN
FLUORESCENT PROBES FOR CHROMOSOMAL PAINTING

(30) Priorité: 15.10.1998 FR 9812957
(43) Date de publication de la demande: 08.08.2001
(73) Titulaire: Serono Genetics Institute S.A., 91030 Evry Cedex (FR)
(72) Inventeur: CHERIF, Dorra, F-75014 Paris (FR)
(74) Mandataire: von Ballmoos, Prisca
(86) Numéro de dépôt international: PCT/FR1999/002517
(87) Numéro de publication internationale: WO 2000/022164

(56) Documents cités:
- WO-A-98/38333
- US-A- 5 817 462
- WILGENBUS, KLAUS KASIMIR ET AL: "IRS-long range (LR) PCR: a simple method for efficient amplification of human genomic DNA from complex sources" METHODS MOL. CELL. BIOL. (1995), VOLUME DATE 1994-1995, 5(4), 214-21, XP002114358
- SPEICHER M R ET AL: "KARYOTYPING HUMAN CHROMOSOMES BY COMBINATORIAL MULTI-FLUOR FISH" NATURE GENETICS, vol. 12, janvier 1996 (1996-01), pages 368-375, XP002900459 ISSN: 1061-4036 cité dans la demande
- LEDBETTER S A ET AL: "RAPID IOSLATION OF DNA PROBES WITHIN SPECIFIC CHROMOSOME REGIONS BY INTERSPERSED REPETITIVE SEQUENCE POLYMERASE CHAIN REACTION" GENOMICS, vol. 6, 1990, pages 475-481, XP002914387 ISSN: 0888-7543
- LICHTER P ET AL: "FLUORESCENCE IN SITU HYBRIDIZATION WITH ALU AND L1 POLYMERASE CHAIN REACTION PROBES FOR RAPID CHARACTERIZATION OF HUMAN CHROMOSOMES IN HYBRID CELL LINES" PROCEEDINGS OF THE NATIONAL ACADEMY OF SCIENCES OF USA, vol. 87, no. 17, 1 septembre 1990 (1990-09-01), pages 6634-6638, XP000310538 ISSN: 0027-8424 cité dans la demande
- LENGAUER ET AL.: "Painting human chromosomes with probes generated from hybrid cell lines by PCR with Alu and L1 primers" HUMAN GENETICS, vol. 86, 1990, pages 1-6, XP002114359
- ROMANA ET AL.: "A simple method for prenatal diagnosis of trisomy 21 on uncultured amniocytes" EUROPEAN JOURNAL OF HUMAN GENETICS, vol. 1, no. 3, 1993, pages 245-251, XP002114368 cité dans la demande
- SCHROECK E ET AL: "MULTICOLOR SPECTRAL KARYOTYPING OF HUMAN CHROMOSOMES" SCIENCE,US,AMERICAN ASSOCIATION FOR THE ADVANCEMENT OF SCIENCE,, vol. 273, 26 juillet 1996 (1996-07-26), pages 494-497, XP002900460 ISSN: 0036-8075 cité dans la demande
- EILS R. ET AL: "An optimized, fully automated system for fast and accurate identification of chromosomal rearrangements by multiplex-FISH (M-FISH)" CYTOGENET CELL GENET, vol. 82, 1998, pages 160-171,

### LISTAGE DE SEQUENCES

<110> GENSET
<120> SONDES FLUORESCENTES DE PEINTURE CHROMOSOMIQUE
<130> 340 291/D.17757/PM
<150> FR 98/12957
   <151> 1998-10-15
<160> 3
<210> 1
   <211> 20
   <212> DNA
   <213> Artificial Sequence
<220>
   <221> primer_bind
   <222> 1..20
   <223> primer PCR Alu
<400> 1
   CCACTGCACT CCAGCCTGGG 20
<210> 2
   <211> 30
   <212> DNA
   <213> Artificial Sequence
<220>
   <221> primer_bind
   <222> 1..30
   <223> primer PCR LINE
<400> 2
   CATGGCACAT GTATACATAT GTAACAAACC 30
<210> 3
   <211> 30
   <212> DNA
   <213> Artificial Sequence
<220>
   <221> primer_bind
   <222> 1..30
   <223> primer PCR LINE
<400> 3
   CATGGCACAT GTATACATAT GTAACTAACC 30

## Description

La présente invention concerne la peinture chromosomique et plus particulièrement les sondes fluorescentes utilisables dans des méthodes telles que la méthode FISH ("Fluorescence *In Situ* Hybridization"). Des combinaisons de fluorophores et de filtres optiques sont également décrites.

L'hybridation *in situ* est une technique permettant de détecter une séquence d'ADN (ou d'ARN) au moyen d'une sonde de séquence spécifique homologue à celle étudiée. Elle est fondée sur la complémentarité des nucléotides (A/T, A/U, G/C) et elle peut être réalisée en conditions physicochimiques précises sur des préparations chromosomiques ou tissulaires. Le résultat du processus d'hybridation *in situ* est la formation d'un hybride entre une sonde et une cible. L'hybridation *in situ* inclut une étape de dénaturation et également une étape de détection de l'hybride ou de la sonde qui est réalisée après l'hybridation *in situ* de la sonde sur la cible. L'échantillon peut adhérer sous forme de couche à la surface de la lame et l'échantillon peut, par exemple, comprendre ou contenir des chromosomes individuels ou des régions chromosomiques qui ont été traités pour maintenir leur morphologies sous des conditions de dénaturation. Dans le cadre de l'hybridation fluorescente *in situ*, les sondes sont marquées avec un fluorophore et l'hybridation est révélée par un marquage fluorescent.

Le développement récent de cette technique permet la visualisation simultanée, sur la même préparation, de plusieurs sondes révélées chacune par un fluorophore différent. Cette technique dénommée FISH-multicouleur ou multi-FISH a été rendue possible par la combinaison de filtres spécifiques des longueurs d'onde d'émission des différentes molécules fluorescentes assurant le marquage grâce à une imagerie assistée par ordinateur réalisée au moyen de caméras CCD refroidies de haute résolution sensibles dans l'infrarouge (Schröck et al., 1996 ; Speicher et al., 1996).

L'utilisation de sondes présentant une séquence spécifique homologue à une séquence chromosomique précise ou un chromosome entier couplé aux potentialités d'un marquage fluorescent multicouleur permet de développer des techniques dites de peinture chromosomique, c'est-à-dire d'obtenir des chromosomes de différentes couleurs et ainsi d'obtenir, si cela est souhaité, un caryotype complet multicouleur. On entend par caryotype, l'arrangement caractéristique des chromosomes d'une cellule à la métaphase.

Au sens général du terme, on entend par "marquage" une entité telle qu'un isotope radioactif ou une entité non isotopique telle que des enzymes, la biotine, l'avidine, la streptavidine, la digoxygénine, les agents luminescents, les colorants, les haptènes et autres. Les agents luminescents, selon la source d'énergie d'excitation, peuvent être classés en radioluminescents, chémiluminescents, bioluminescents et photo-luminescents (incluant fluorescents et phosphorescents). Le terme "fluorescent" se réfère en général à la propriété d'une substance (telle un fluorophore) de produire de la lumière lorsqu'elle est excitée par une source énergétique telle que la lumière ultra-violette par exemple.

On entend par "sonde de peinture chromosomique" une sonde ou une composition de sondes telle la composition de sondes de cette invention, qui est adaptée pour hybrider, sous les conditions d'hybridation, avec une cible qui comprend un chromosome prédéterminé d'un génome multi-chromosomique. Si seulement une fraction d'un tel chromosome est présente dans l'échantillon subissant une telle hybridation avec une telle composition de sondes, alors cette fraction s'hybride et est identifiée. En pratique, une sonde peinte de cette invention peut être mélangée avec une seconde, une troisième, etc. pour permettre le marquage et la détection simultanée des deux, des trois, etc. chromosomes prédéterminés.

La visualisation de l'ensemble des 24 chromosomes humains a été rendue possible par l'utilisation d'un marquage avec une combinaison de fluorochromes. Par exemple, dans le cas de l'utilisation de 5 fluorophores différents, 31 combinaisons de fluorophores peuvent être obtenues. En utilisant ce principe de marquage et 24 sondes d'ADN spécifiques de chacun des chromosomes humains, il a été possible de visualiser chaque chromosome de façon différentielle. L'attribution, par le traitement informatique, de couleurs artificielles à chacune des combinaisons de fluorophores permet ainsi de colorer différemment les 24 chromosomes humains.

Rapidement, les fortes potentialités d'un tel marquage multicouleur ont permis l'analyse d'aberrations chromosomiques jusqu'alors difficilement détectables par les techniques cytogénétiques classiques de marquage des chromosomes en bandes (Summer et al., 1971 ; Dutrillaux et Lejeune, 1971) (coloration au Giemsa, marquage au BrdU, etc.). Le principe de marquage des chromosomes en bandes repose sur les différences dans la composition moyenne en paires de base (richesse en GC) entre les bandes et sur les différences de compaction de la chromatine entre les bandes chromosomiques. La peinture chromosomique s'avère être un outil très utile pour détecter les aberrations interchromosomiques telles que les translocations, les séquences d'ADN amplifiées telles les régions colorées de façon homogène appelées HSR (HSR pour Homogeneously Staining Regions) ou les excès de matériels chromosomiques tels que les chromosomes marqueurs ou des chromosomes double-minute. Les aberrations intrachromosomiques telles que les délétions et duplications ne seront détectées qu'en fonction de la taille des aberrations, si celles-ci affectent la longueur des chromosomes, alors que les inversions chromosomiques ne seront pas du tout détectables par cette méthode.

Les limites d'utilisation du caryotypage spectral actuel en tant que tel sont dues au fait qu'il ne permet pas de détecter la nature des bandes chromosomiques impliquées dans un remaniement, inter- ou intrachromosomique. Pour ce faire, il est indispensable de coupler cette technique à celle plus conventionnelle des bandes chromosomiques (marquage R ou G) telle la contre-coloration au DAPI, la coloration au Giemsa ou à l'iodure de propidium par exemple.

L'obligation de combiner différentes techniques constitue bien entendu un handicap dans l'analyse des aberrations chromosomiques et, par ailleurs, l'utilisation de la méthode FISH ou multi-FISH qui associe le prix élevé de l'appareillage et de l'instrumentation nécessaire à la visualisation de la peinture chromosomique au prix élevé des sondes spécifiques des chromosomes restreint les possibilités de diffusion de cette technique auprès des laboratoires de recherche ou des laboratoires de diagnostic.

Des sondes de peinture actuellement disponibles sur le marché (GIBCO-BRL, Oncor, Bochringer Manheim et autres) sont obtenues par amplification DOP-PCR utilisant des amorces PCR dégénérées de chromosomes ou de fragments de chromosomes isolés par des techniques lourdes telle le tri chromosomique par cytométrie de flux ou la microdissection de chromosomes. L'hybridation de sondes obtenues par DOP-PCR ne génère pas de bandes chromosomiques sur les chromosomes. La génération de bandes chromosomiques a été recherchée par l'intermédiaire de la création de bandes artificielles le long de chromosomes. Cette création de bandes artificielles nécessite l'utilisation de techniques lourdes et coûteuses. De plus, elle aboutit à des bandes qui ne sont pas des repères connus dans le domaine de la cytogénétique.

Certains auteurs ont décrit l'utilisation de sondes de peinture chromosomique obtenue par amplification de chromosomes par IRS-PCR (Interspersed Repeatcd Sequences) utilisant des amorces spécifiques des séquences d'ADN répétées et dispersées dans le génome comme les séquences Alu et LINE. L'utilisation séparée d'amorces PCR LINE ou Alu pour obtenir des sondes de peinture chromosomique a été proposée par Lenguauer et al., 1990. L'utilisation combinée d'amorces PCR LINE et Alu pour l'amplification de chromosomes humains par IRS-PCR a été proposée au préalable par Lichter at al., 1990. Néanmoins, le marquage en bandes R obtenu par ces derniers ne permet pas d'assurer une peinture complète couvrant toutes les régions du génome, notamment les régions télomériques et certaines bandes chromosomiques G.

La présente invention a pour objet de fournir des mélange de sondes chromosomiques qui peuvent être obtenues de façon peu onéreuse et qui en outre permettent de faire apparaître directement les bandes chromosomiques de qualité sur les chromosomes peints dans leur totalité.

Pour ce faire, la présente invention concerne des mélange de sondes destinées au marquage d'un chromosome, caractérisées en ce qu'elles sont composées d'un ensemble de segments d'ADN davantage représentés dans certaines bandes chromosomiques et obtenus par amplification IRS-PCR à partir desdits chromosomes à l'aide d'amorces spécifiques des séquences d'ADN Alu et LINE.

Le terme "sonde" se réfère à un polynucléotide ou un mélange de polynucléotides tels que des segments d'ADN ou des séquences d'ADN sont associés chimiquement à des entités individuelles marquées. Chacun des polynucléotides composant une sonde est de manière caractéristique sous forme simple brin au moment de l'hybridation sur la cible.

Le terme "fragment d'ADN", "segment d'ADN" indique généralement seulement une portion d'un polynucléotide ou d'une séquence présente dans un chromosome ou une portion de chromosome. Un polynucléotide par exemple peut être coupé ou fragmenté en une multitude de segments ou de fragments. Un chromosome contient de manière caractéristique des régions qui ont des séquences d'ADN contenant des segments d'ADN répétés. Le terme "répété" se réfère au fait qu'un segment d'ADN particulier est présent de nombreuses fois (au moins deux fois) de manière dispersée ou non dans le génome. La méthode dite IRS-PCR utilise des amorces hybridant avec les séquences répétées dispersées du génome, comme, par exemple, les séquences Alu ou LINE.

On désigne par "génome" la copie complète et unique des instructions génétiques d'un organisme codées par l'ADN de cet organisme. Dans la présente invention, le génome particulier considéré est multichromosomique de telle sorte que l'ADN est distribué dans la cellule entre plusieurs chromosomes individuels. Le génome humain se compose de 23 paires de chromosomes dont une paire XX ou XY déterminant le sexe.

Le terme "chromosome" se réfère au support des gènes porteurs de l'hérédité dans une cellule vivante qui dérive de la chromatine et qui comprend de l'ADN et des composants protéiques (essentiellement les histones). Le système conventionnel international d'identification et de numérotation des chromosomes du génome humain est employé ici. La taille d'un chromosome individuel peut varier dans un génome multi-chromosomique et d'un génome à l'autre. Dans le cas présent du génome humain, la longueur totale d'ADN d'un chromosome donné est généralement supérieure à 50 000 000 pb. A titre de comparaison, la longueur totale du génome humain est de 3.10 ⁹ pb.

Le génome des mammifères contient des séquences répétées d'ADN dispersées sur tout le génome. Chez l'homme, la majeure partie de ce type de séquences est représentée par les différentes familles de séquences Alu, qui sont au nombre de 10⁶ environ, et ont en commun une séquence consensus de 300 pb. Les séquences répétées LINES (ou L1) sont, comme les séquences Alu, largement distribuées sur tout le génome. Elles sont cependant moins nombreuses (environ 10⁴). Leur séquence consensus est d'environ 6 kb. Elles sont préférentiellement situées dans les bandes sombres G (G positives ou R négatives), alors que les séquences Alu sont plutôt situées dans les bandes sombres R (R positives) (Korenberg et Kirowski, 1988).

Les segments d'ADN amplifiés par IRS-PCR selon l'invention peuvent avoir pour source des hybrides somatiques, de préférence rongeur-homme, des chromosomes ou des fragments de chromosome. Les chromosomes ou fragments de chromosome peuvent être obtenus par tri chromosomique par cytométrie de flux ou par microdissection chromosomique. Dans le cadre du mode préféré de réalisation de la présente invention, les segments d'ADN amplifiés par IRS-PCR ont pour source des hybrides somatiques rongeur-homme mono-chromosomiques.

Les segments d'ADN amplifiés par IRS-PCR selon l'invention sont davantage représentés dans un type de bandes cytogénétiques. Les bandes cytogénétiques préférées sont les bandes G ou les bandes R. Dans le mode préféré de réalisation de la présente invention, les segments d'ADN amplifiés par IRS-PCR sont davantage représentés en bandes R.

Des séquences répétées, analogues aux séquences répétées humaines, sont aussi retrouvées dans le génome des rongeurs. Toutefois la divergence de ce type de séquences entre l'homme et le rongeur est suffisamment importante pour qu'il y ait peu d'homologie entre elles. Cette divergence permet une amplification sélective de segments d'ADN contenus dans le chromosome humain lorsque des hybrides homme-rongeur sont utilisés. Ainsi en partant de l'ADN d'un hybride somatique homme-rongeur et en utilisant des amorces spécifiques de la séquence consensus Alu et/ou L1, on peut amplifier sélectivement par PCR les séquences d'ADN comprises entre deux séquences répétées (en position "tête-bêche") séparées par une distance < 5 kb. Le produit d'amplification ainsi obtenu est constitué d'un ensemble de fragments (dont la taille varie environ de 100 pb à 5 kb) représentatif de la quasi totalité du chromosome humain contenu dans l'ADN de l'hybride somatique.

De façon générale, la présente invention est bien entendu plus particulièrement destinée à réaliser des mélanges de sondes spécifiques de chromosomes humains, même s'il est possible d'envisager des peintures chromosomiques pour d'autres types cellulaires (Sabile et al., 1997).

Les mélanges de sondes de la présente invention sont caractérisées en ce que les sondes sont issues d'un mélange de deux produits d'amplification IRS-PCR composé d'une part d'un:
- produit d'amplification PCR obtenu par l'utilisation de l'amorce spécifique des séquences d'ADN Alu,
et d'autre part d'un:
- produit d'amplification PCR obtenu par l'utilisation de l'amorce spécifique des séquences d'ADN Alu et de l'amorce spécifique des séquences d'ADN LINE.

La présente invention concerne un procédé de production de mélanges de sondes destinées au marquage de chromosomes humains, caractérisé en ce que ledit procédé comprend le mélange de deux produits d'amplification obtenus par deux amplifications IRS-PCR à partir desdits chromosomes en utilisant d'une part des amorces PCR spécifiques des séquences d'ADN Alu et LINE, et d'autre part des amorces PCR spécifiques des séquences d'ADN Alu.

Toute amorce spécifique des séquences Alu ou LINE peut être utilisée dans la présente invention. De préférence, les amorces spécifiques des séquences d'ADN Alu sont constituées par l'amorce SR1 dont la séquence est décrite dans la SEQ ID No 1 et l'amorce spécifique de la séquence d'ADN LINE est de préférence l'amorce L1H dont la séquence est décrite dans les SEQ ID Nos 2 et 3.

De manière alternative, des mélanges de sondes selon la présente invention peuvent également être issues d'un mélange de deux produits d'amplification IRS-PCR composé d'une part d'un:
- produit d'amplification PCR obtenu par l'utilisation de l'amorce spécifique des séquences d'ADN LINE,
et d'autre part d'un:
- produit d'amplification PCR obtenu par l'utilisation de l'amorce spécifique des séquences d'ADN Alu et de l'amorce spécifique des séquences d'ADN LINE.

La présente invention concerne également un procédé de production de mélanges de sondes destinées au marquage de chromosomes humains, caractérisé en ce que ledit procédé comprend le mélange de deux produits d'amplification obtenus par deux amplifications IRS-PCR à partir desdits chromosomes en utilisant d'une part des amorces PCR spécifiques des séquences d'ADN Alu et LINE, et d'autre part des amorces PCR spécifiques des séquences d'ADN LINE.

L'utilisation de fluorophores et de filtres dont la combinaison permet d'assurer une peinture chromosomique fournissant des caryotypes très lisibles, c'est-à-dire d'obtenir des couleurs de peinture chromosomique contrastées et de bonne définition est également décrite.

Ainsi, les sondes d'ADN décrites précédemment sont marquées directement ou indirectement par des techniques de fluorescence. De manière non exhaustive, les fluorophores utilisés pour le marquage peuvent être choisis parmi les marqueurs de type cyanine, rhodamine, fluorescéine, Bobipy, Rouge Texas, Vert Oregon, Bleu Cascade. Notamment, tous les fluorophores cités dans le « Handbook of fluorescent probes and research chemicals » (Richard P Haugland, 1996, Molecular Probes, MTZ Spence Ed., plus particulièrement p 145-146, 153, 155-156, 157-158, 161) peuvent être utilisés pour marquer les sondes de la présente invention.

De préférence, les sondes selon l'invention sont marquées par au moins 1, 2, 3, 4, ou 5 fluorophores choisis parmi le groupe suivant : isothiocyanate de fluoresceine (FITC), Rouge Texas (TR pour Texas Red), cyanine 3 (Cy3), cyanine 5 (Cy5), cyanine 5,5 (Cy5,5), cyanine 7 (Cy7), Bodipy 630/650.

La méthode préférée de marquage des sondes d'ADN est la « Nick translation ». Néanmoins, le marquage peut être également effectué selon toutes les réactions standards de synthèse d'ADN catalysée par une polymérase et de marquage d'oligonucléotides. Par exemple, le marquage peut être réalisé par les techniques d'amorçage aléatoire, d'amplification, d'extension d'amorce in situ.

Le terme "sonde directement marquée" désigne ou décrit une sonde d'acide nucléique dont le marquage après la formation d'hybride avec la cible est détectable sans traitement réactif subséquent de l'hybride. Les sondes utilisant les fluorophores FITC, Rouge Texas, Cy3 et Cy5 selon la présente invention sont directement marquées.

Le terme "sonde indirectement marquée" désigne ou décrit une sonde d'acide nucléique dont le marquage après la formation d'hybride avec la cible doit subir un traitement réactif supplémentaire avec un ou plusieurs réactifs pour y associer une ou plusieurs entités dont résulte(nt) finalement un composé détectable. Par exemple, les sondes peuvent être marquées par le DNP, la digoxigénine ou la biotine et la révélation comprend la mise en contact de la sonde avec un anticorps anti-DNP ou anti-digoxigénine marqué par un fluorophore ou avec une avidine couplée à un fluorophore. Les sondes utilisant les fluorophores Cy7, Bodipy 630/650, Cy 5.5 selon la présente invention sont indirectement marquées.

De préférence, la composition en sondes de la présente invention comprend le plus grand nombre possible de sondes « directement marquées ». Outre que les sondes directement marquées sont d'un usage plus facile, elles permettent une meilleure résolution. Cette bonne résolution est importante pour une bonne observation des bandes chromosomiques. De préférence, la composition en sondes de la présente invention est de type « marquage direct » pour tous les fluorophores. Une composition en sonde préférée de la présente invention est de type "marquage direct" pour 4 des 5 fluorophores utilisés. Dans une autre composition préférée, les sondes directement marquées représentent 3 sondes sur 5 ou 6 fluorophores utilisés.

La présente invention concerne également un ensemble de mélanges de sondes destinées au marquage de chromosomes humains, caractérisées en ce qu'il contient des mélanges de sondes selon la présente invention pour chacun des chromosomes humains ou pour un certain nombre d'entre eux. Cet ensemble de mélanges de sondes permettra d'analyser en une seule fois un caryotype complet afin d'y détecter et d'y identifier d'éventuelles aberrations chromosomiques telles que décrites précédemment.

La présente invention concerne également un procédé FISH multicouleur destiné à l'étude du caryotype, caractérisé en ce que les sondes d'ADN sont marquées par des fluorophores et en ce que chaque fluorophore de longueur d'onde d'absorption et d'émission spécifique est associé à un couple de filtres optiques, un pour l'absorption et un pour l'émission.

Les fluorophores sont choisis de telle sorte que le chevauchement des spectres d'absorption et d'émission entre les différents fluorophores soit minimal. Plus particulièrement, il est important qu'il n'y aie pas de chevauchement entre les maxima d'absorption et d'émission dès différents fluorophores.

Chaque fluorophore est utilisé avec un couple de filtres optiques ; un pour l'absorption et un pour l'émission. Les filtres permettent de sélectionner des bandes passantes de telles sortes que les longueurs d'onde correspondant à un chevauchement avec un autre fluorophore soit éliminées. C'est pourquoi les filtres utilisés dans la présente invention sont de préférence du type filtre optique à bande passante étroite. Les filtres sont de préférence de qualité supérieure car il est important que le filtre ne laisse pas passer de lumière en dehors de la bande passante.

De préférence, la présente invention concerne également un procédé FISH multicouleur destiné notamment à l'étude du caryotype, caractérisé en ce que les sondes d'ADN selon la présente invention sont marquées par des fluorophores et en ce que chaque fluorophore de longueur d'onde d'absorption et d'émission spécifique est associé à un couple de filtres optiques, un pour l'absorption et un pour l'émission, ledit procédé utilisant des fluorophores et des couples de filtres choisis parmi le groupe suivant :
a) le fluorophore FITC ayant une longueur d'onde maximale d'absorption de 494 nm et une longueur d'onde maximale d'émission de 517 nm associé à un filtre à l'excitation de type 490DF30 (Omega Optical) et à un filtre à l'émission de type 530DF30 (Omega Optical),
b) le fluorophore Cy3 ayant une longueur d'onde maximale d'absorption de 554 nm et une longueur d'onde maximale d'émission de 568 nm associé à un filtre à l'excitation de type 546DF10 (Omega Optical) et à un filtre à l'émission de type 570DF10 (Omega Optical),
c) le fluorophore TR ayant une longueur d'onde maximale d'absorption de 593 nm et une longueur d'onde maximale d'émission de 613 nm associé à un filtre à l'excitation de type 590DF10 (Omega Optical) et à un filtre à l'émission de type 615DF10 (Omega Optical),
d) le fluorophore Cy5 ayant une longueur d'onde maximale d'absorption de 652 nm et une longueur d'onde maximale d'émission de 670 nm associé à un filtre à l'excitation de type 650DF20 (Omega Optical) et à un filtre à l'émission de type 670DF10 (Omega Optical),
e) le fluorophore Cy7 ayant une longueur d'onde maximale d'absorption de 743 nm et une longueur d'onde maximale d'émission de 767 nm associé à un filtre à l'excitation de type 740DF25 (Omega Optical) et à un filtre à l'émission de type 780EFLP (Omega Optical),
f) le fluorophore Cy5,5 ayant une longueur d'onde maximale d'absorption de 675 nm et une longueur d'onde maximale d'émission de 694 nm associé à un filtre à l'excitation de type 680DF20 (Omega Optical) et à un filtre à l'émission de type 700EFLP (Omega Optical),
g) le fluorophore Bodipy 630/650 ayant une longueur d'onde maximale d'absorption de 632 nm et une longueur d'onde maximale d'émission de 658 nm associé à un filtre à l'excitation de type 630DF20 (Omega Optical) et à un filtre à l'émission de type 650DF10 (Omega Optical).

L'invention concerne également un kit de diagnostic FISH multicouleur caractérisé en ce qu'il comprend des mélanges de sondes d'ADN selon la présente invention marquées par des fluorophores et en ce que chaque fluorophore de longueur d'onde d'absorption et d'émission spécifique est associé à un couple de filtres optiques, un pour l'absorption et un pour l'émission, ledit kit utilisant des fluorophores et des couples de filtres choisis parmi le groupe suivant :
a) le fluorophore FITC ayant une longueur d'onde maximale d'absorption de 494 nm et une longueur d'onde maximale d'émission de 517 nm associé à un filtre à l'excitation de type 490DF30 (Omega Optical) et à un filtre à l'émission de type 530DF30 (Omega Optical),
b) le fluorophore Cy3 ayant une longueur d'onde maximale d'absorption de 554 nm et une longueur d'onde maximale d'émission de 568 nm associé à un filtre à l'excitation de type 546DF10 (Omega Optical) et à un filtre à l'émission de type 570DF10 (Omega Optical),
c) le fluorophore TR ayant une longueur d'onde maximale d'absorption de 593 nm et une longueur d'onde maximale d'émission de 613 nm associé à un filtre à l'excitation de type 590DF10 (Omega Optical) et à un filtre à l'émission de type 615DF10 (Omega Optical),
d) le fluorophore Cy5 ayant une longueur d'onde maximale d'absorption de 652 nm et une longueur d'onde maximale d'émission de 670 nm associé à un filtre à l'excitation de type 650DF20 (Omega Optical) et à un filtre à l'émission de type 670DF10 (Omega Optical),
e) le fluorophore Cy7 ayant une longueur d'onde maximale d'absorption de 743 nm et une longueur d'onde maximale d'émission de 767 nm associé à un filtre à l'excitation de type 740DF25 (Omega Optical) et à un filtre à l'émission de type 780EFLP (Omega Optical),
f) le fluorophore Cy5,5 ayant une longueur d'onde maximale d'absorption de 675 nm et une longueur d'onde maximale d'émission de 694 nm associé à un filtre à l'excitation de type 680DF20 (Omega Optical) et à un filtre à l'émission de type 700EFLP (Omega Optical),
g) le fluorophore Bodipy 630/650 ayant une longueur d'onde maximale d'absorption de 632 nm et une longueur d'onde maximale d'émission de 658 nm associé à un filtre à l'excitation de type 630DF20 (Omega Optical) et à un filtre à l'émission de type 650DF10 (Omega Optical).

Les filtres selon la présente invention sont de préférence tels :
- qu'ils soient de type 6 cavités,
- qu'ils aient une ADI de 0°;
- qu'ils aient une tolérance λ₀ ± 20% de FWHM,
- qu'ils aient une tolérance sur FWHM de ± 20% de FWHM,
- qu'ils aient une réjection hors bande passante OD5 de UV à 1200 nm
- qu'ils aient une courbe de transmission T ≥ 50% à λ₀.

De préférence, les filtres doivent également avoir un diamètre utile centré supérieur à 21 mm, et une épaisseur ≤ 7 mm,

Les fluorophores et les filtres précédents peuvent être utilisés pour le marquage des sondes selon la présente invention ou bien pour des sondes différentes utilisées, par exemple pour la peinture chromosomique ou pour le FISH multicouleur.

Dans la présente invention, on entend par "filtres" des filtres interférentiels à bande passante étroite qui transmettent la lumière à l'intérieur d'une bande spectrale donnée très étroite, centrée autour de la longueur d'onde λ₀ de référence. Ils sont caractérisés par leur courbe de transmission : T = f(λ). La largeur de la bande est définie par la largeur totale à la moitié du maximum de transmission (FWHM pour "Full Width at Half Maximum transmission").

En dehors de la bande passante, le filtre laisse passer un signal résiduel qui a tout intérêt à être le plus atténué possible.

Les filtres interférentiels fonctionnent sur le principe des interférences constructives et destructives. La composante de base d'un filtre interférentiel est appelée cavité. Elle comporte deux piles de réflecteurs séparées par une couche d'un solide diélectrique. Plus le nombre de cavités est important, plus la forme de la courbe de transmission est rectangulaire (c'est-à-dire, plus la pente de cette courbe est importante). D'autre part, plus le nombre de cavités est important, meilleur est le coefficient d'atténuation en dehors de la bande passante.

Pour l'application de multifluorescence, les spectres d'excitation ou d'émission des fluorochromes utilisés sont très proches les uns des autres. Il faut donc récupérer le minimum de signal possible en dehors de la bande passante. C'est pourquoi, on a choisi des filtres à 6 cavités qui offrent les meilleures caractéristiques à ce niveau.

Les filtres utilisés ont de préférence les spécifications suivantes :
- ils sont conçus pour être utilisés en incidence normale de la lumière,
- la tolérance sur la longueur d'onde centrale (λ₀) est de ± 20% de la bande passante, par exemple, pour un filtre de bande passante de 10 nm, λ₀ sera définie avec une tolérance de ± 2 nm,
- la tolérance sur la largeur de la bande passante est de ± 20%,
- le coefficient de transmission T de ces filtres est supérieur à 50%,
- la réjection hors bande passante de ces filtres est de 5OD de l'ultra-violet à 1200 nm, ceci signifie qu'en dehors de la bande passante, le coefficient de transmission est de 10⁻⁵, soit 0,001%. Pour des filtres standards, la réjection hors bande passante est assurée pour des longueurs d'onde allant de 0,8 λ₀ à 1,2 λ₀, par exemple, pour un filtre de λ. = 620 nm, la réjection hors bande passante se fait seulement entre 500 et 740nm. Or, pour l'application de multifluorescence, on observe des fluorochromes dont les spectres s'étendent de 350 à 800 nm. C'est pourquoi on a utilisé des filtres dont la réjection hors bande passante est assurée de l'ultra-violet à 1200 nm.

Un kit de marquage caractérisé en ce qu'il comprend au moins des mélanges de sondes d'ADN telles que décrites précédemment ou un ensemble de sondes tel que mentionné précédemment, est également décrit.

La présente in ention concerne un kit de diagnostic FISH multicouleur, caractérisé en ce qu'il comprend les mélanges de sondes d'ADN telles que décrites précédemment ou un ensemble de sondes d'ADN tel que mentionné précédemment et une combinaison de filtres et de fluorophores tels que décrits précédemment.

La technique FISH ou multi-FISH à laquelle il est ou il sera fait allusion à plusieurs reprises dans la présente description est notamment décrite dans Speicher et al., 1996 ; Schröck et al., 1996.

D'autres caractéristiques et avantages de la présente invention apparaîtront à la lecture des exemples ci-après.

Les combinaisons de fluorophores et de filtres optiques décrites peuvent être utilisées dans de multiples techniques impliquant la microscopie de fluorescence. En effet, les fluorophores décrits peuvent être utilisés pour marquer de nombreuses molécules ou structures. De manière non exhaustive, lesdits fluorophores peuvent être utilisés pour marquer des polypeptides, des anticorps, des acides nucléiques, des phospholipides, des acides gras, des dérivés stéroles, des membranes, des organelles et beaucoup d'autres macromolécules biologiques. Les organelles peuvent être les mitochondries, le réticulum endoplasmique, l'appareil de Golgi, et les lysosomes.

La combinaison de fluorophores et de filtres optiques peut être utilisée pour effectuer du FISH. Notamment, cela peut permettre l'utilisation simultanée de plusieurs sondes. Cette combinaison peut être utilisée pour étudier de multiples aspects comme la morphologie cellulaire, le cytosquelette, les récepteurs cellulaires, les canaux ioniques, les neurotransmetteurs, la circulation des fluides, la fluidité membranaire, la viabilité et la prolifération cellulaire, l'apoptose, la pinocytose, l'endocytose et l'exocytose, la transduction, le pH et les concentrations ioniques (par exemple de calcium, potassium, magnésium et zinc) (Richard P Haugland, 1996, Molecular Probes, MTZ Spence Ed.). Elle peut permettre d'étudier l'expression et la traduction.

Une combinaison de fluorophores choisi parmi : isothiocyanate de fluoresceine (FITC), Rouge Texas (TR pour Texas Red), cyanine 3 (Cy3), cyanine 5 (Cy5), cyanine 5,5 (Cy5,5), cyanine 7 (Cy7), Bodipy 630/650, est décrite.

De préférence, la combinaison de fluorophores comprend au moins 2, 3, 4, 5, 6 ou 7 fluorophores choisis parmi : isothiocyanate de fluoresceine (FITC), Rouge Texas (TR pour Texas Red), cyanine 3 (Cy3), cyanine 5 (Cy5), cyanine 5,5 (Cy5,5), cyanine 7 (Cy7), Bodipy 630/650.

Une combinaison de fluorophores préférée comprend les 5 fluorophores suivants : l'isothiocyanate de fluoresceine (FITC), le rouge Texas (TR), la cyanine 3 (Cy3), la cyanine 5 (Cy5), et la cyanine 7 (Cy7).

Une autre combinaison de fluorophores préférée comprend les 6 fluorophores suivants : l'isothiocyanate de fluoresceine (FITC), le rouge Texas (TR), la cyanine 3 (Cy3), le Bodipy 630/650, la cyanine 5 (Cy5), et la cyanine 7 (Cy7).

Une autre combinaison de fluorophores préférée comprend 5 fluorophores suivants : l'isothiocyanate de fluoresceine (FITC), le rouge Texas (TR), la cyanine 3 (Cy3), le Bodipy 630/650, et la cyanine 5 (Cy5).

Les combinaisons de fluorophores peuvent être comprises dans un kit de diagnostic FISH multicouleur.

Les combinaisons de fluorophores peuvent être utilisées pour marquer une entité choisie parmi les polypeptides, les anticorps, les acides nucléiques, les phospholipides, les acides gras, les dérivés stéroles, les membranes, les organelles et les macromolécules biologiques

En outre, il est décrit une combinaison de fluorophores associés à un couple de filtres optiques choisis parmi :
a.le fluorophore FITC ayant une longueur d'onde maximale d'absorption de 494 nm et une longueur d'onde maximale d'émission de 517 nm associé à un filtre à l'excitation de type 490DF30 (Omega Optical) et à un filtre à l'émission de type 530DF30 (Omega Optical),
b. le fluorophore Cy3 ayant une longueur d'onde maximale d'absorption de 554 nm et une longueur d'onde maximale d'émission de 568 nm associé à un filtre à l'excitation de type 546DF10 (Oméga Optical) et à un filtre à l'émission de type 570DF10 (Omega Optical),
c. le fluorophore TR ayant une longueur d'onde maximale d'absorption de 593 nm et une longueur d'onde maximale d'émission de 613 nm associé à un filtre à l'excitation de type 598DF10 (Omega Optical) et à un filtre à l'émission de type 615DF10 (Omega Optical),
d. le fluorophore Cy5 ayant une longueur d'onde maximale d'absorption de 652 nm et une longueur d'onde maximale d'émission de 670 nm associé à un filtre à l'excitation de type 650DF20 (Omega Optical) et à un filtre à l'émission de type 670DF10 (Omega Optical),
e. le fluorophore Cy7 ayant une longueur d'onde maximale d'absorption de 743 nm et une longueur d'onde maximale d'émission de 767 nm associé à un filtre à l'excitation de type 740DF25 (Omega Optical) et à un filtre à l'émission de type 780EFLP (Omega Optical),
f. le fluorophore Cy5,5 ayant une longueur d'onde maximale d'absorption de 675 nm et une longueur d'onde maximale d'émission de 694 nm associé à un filtre à l'excitation de type 680DF20 (Omega Optical) et à un filtre à l'émission de type 700EFLP (Omega Optical),
g. le fluorophore Bodipy 630/650 ayant une longueur d'onde maximale d'absorption de 632 nm et une longueur d'onde maximale d'émission de 658 nm associé à un filtre à l'excitation de type 630DF20 (Omega Optical) et à un filtre à l'émission de type 650DF10 (Omega Optical).

De préférence, la combinaison de fluorophores associés à un couple de filtres optiques comprend au moins 1, 2, 3, 4, 5, 6 ou 7 fluorophores et filtres choisis parmi :
a) le fluorophore FITC ayant une longueur d'onde maximale d'absorption de 494 nm et une longueur d'onde maximale d'émission de 517 nm associé à un filtre à l'excitation de type 490DF30 (Omega Optical) et à un filtre à l'émission de type 530DF30 (Omega Optical),
b) le fluorophore Cy3 ayant une longueur d'onde maximale d'absorption de 554 nm et une longueur d'onde maximale d'émission de 568 nm associé à un filtre à l'excitation de type 546DF10 (Omega Optical) et à un filtre à l'émission de type 570DF10 (Omega Optical),
c) le fluorophore TR ayant une longueur d'onde maximale d'absorption de 593 nm et une longueur d'onde maximale d'émission de 613 nm associé à un filtre à l'excitation de type 590DF10 (Omega Optical) et à un filtre à l'émission de type 615DF10 (Omega Optical),
d) le fluorophore Cy5 ayant une longueur d'onde maximale d'absorption de 652 nm et une longueur d'onde maximale d'émission de 670 nm associé à un filtre à l'excitation de type 650DF20 (Omega Optical) et à un filtre à l'émission de type 670DF10 (Omega Optical),
e) le fluorophore Cy7 ayant une longueur d'onde maximale d'absorption de 743 nm et une longueur d'onde maximale d'émission de 767 nm associé à un filtre à l'excitation de type 740DF25 (Omega Optical) et à un filtre à l'émission de type 780EFLP (Omega Optical),
f) le fluorophore Cy5,5 ayant une longueur d'onde maximale d'absorption de 675 nm et une longueur d'onde maximale d'émission de 694 nm associé à un filtre à l'excitation de type 680DF20 (Omega Optical) et à un filtre à l'émission de type 700EFLP (Omega Optical),
g) le fluorophore Bodipy 630/650 ayant une longueur d'onde maximale d'absorption de 632 nm et une longueur d'onde maximale d'émission de 658 nm associé à un filtre à l'excitation de type 630DF20 (Omega Optical) et à un filtre à l'émission de type 650DF10 (Omega Optical).

Les combinaisons de fluorophores associés au couple de filtres optiques peuvent être comprises dans un kit de diagnostic FISH multicouleur.

Les combinaisons de fluorophores associés à un couple de filtres optiques peuvent être utilisées pour marquer une entité choisie parmi les polypeptides, les anticorps, les acides nucléiques, les phospholipides, les acides gras, les dérivés stéroles, les membranes, les organelles et les macromolécules biologiques

Les sondes et les combinaisons de fluorophores peuvent être utilisées avec tout type de microscopes (microscope à fluorescence, laser, monochromateur). De préférence, l'invention utilise un microscope à fluorescence.

### EXEMPLES

### 1. Préparation des sondes

L'ADN génomique extrait de différentes lignées hybrides somatiques homme-rongeur (NIGMS Human genetic Mutant Cell Repository, Coriell Institute for Medical Research, Camdem) (tableau 1) a servi de matrice pour la PCR.

**Tableau 1**

| **Chromosome** | **Référence lignée** |
|---|---|
| 1 | GM 13139 |
| 2 | GM 10826B |
| 3 | GM 10253 |
| 4 | GM 10115 |
| 5 | GM 10114 |
| 6 | GM 11580 |
| 7 | GM 10791 |
| 8 | GM 10156C |
| 9 | GM 10611 |
| 10 | GM 10926B |
| 11 | GM10927A |
| 12 | GM 10868 |
| 13 | GM 10898 |
| 14 | GM 11535 |
| 15 | GM 11715 |
| 16 | GM 10567 |
| 17 | GM 10498 |
| 18 | GM 11010 |
| 19 | GM 10449 |
| 20 | GM 13260 |
| 21 | GM 10323 |
| 22 | GM 10888 |
| X | GM 6318B |

La PCR a été réalisée soit en présence uniquement de l'amorce SR1 (située à l'extrémité 3' de la séquence consensus Alu, position 241 à 261 : 5' CCACTGCACTCCAGCCTGGG 3' (SEQ ID N° 1) (Romana et al., 1993), soit en présence de l'amorce SR1 et de l'amorce L1H:

5' CATGGCACATGTATACATATGTAAC(A/T)AACC 3' (SEQ ID N° 2 et N 3) (Ledbetter et al., 1990). Lorsqu'on a utilisé au cours de la PCR uniquement l'amorce SR1, le produit d'amplification marqué et utilisé comme sonde sur chromosomes métaphasiques a été coloré presque totalement le chromosome correspondant (à l'exception des régions centromériques) avec un profil de bandes de type R (comme cela a été décrit par Lichter et al., 1990 avec d'autres types d'amorces Alu). Cependant, afin d'avoir une représentation des bandes R négatives, on a effectué également une PCR en incorporant les 2 amorces : SR1 et L1. Ainsi, lorsque les 2 produits d'amplification (SR1 et SR1/L1) ont été mélangés et utilisés comme sonde, les bandes R négatives ont bien été colorées et les régions télomériques ont été parfaitement délimitées.

### Conditions de PCR

La réaction de PCR a eu lieu dans un volume final de 50 µl contenant 500 ng d'ADN génomique (hybride somatique), 1 µM de chaque oligonucléotide (soit 1 µM SR1, soit 1 µM SR1 et 1 µM L1H), 10 mM Tris-HCl, 50 mM KCI, 2,5 mM MgCl₂, 0,01% gélatine, 250 µM de chaque déoxynucléotide triphosphate (dATP, dGTP, dCTP, dTTP) et 2,5 U d'ADN-polymérase *Thermophilus aquaticus* (Perkin-Elmer-Cetus). La dénaturation initiale a été effectuée à 96°C pendant 6 min., suivie de 30 cycles : dénaturation à 94°C pendant 1 min., association à 63°C pendant 1 min., élongation à 72°C pendant 10 min. A la fin des cycles, une élongation finale à 72°C pendant 10 min. a été réalisée.

Les 2 produits d'amplification (SR1 et SR1/L1H) ont été mélangés puis précipités à l'éthanol. Le culot d'ADN a été repris dans 20 µl d'eau et la concentration d'ADN a été estimée sur un gel d'agarose 1,3%.

### Marquage des sondes par "Nick translation"

15 µg du mélange de produits PCR ont pu être marqués au cours d'une seule réaction de Nick translation. La réaction a eu lieu dans un volume final de 500 µl, contenant 20 µM de chacun des déoxynucléotides triphosphates (dATP, dGTP, dCTP), 10 µM de dTTP et 10 µM de dUTP modifié, Tris-HCl 50 µM, MgCl₂ 5 µM, β-mercaptoéthanol 1 µM et 20 U de mélange d'enzymes (Dnase/Polymérase : Boehringer-Mannheim). Pour marquer directement la sonde en fluorescence le nucléotide modifié a été soit du dUTP-12-FITC (Boehringer-Mannheim) ou dUTP-12-Texas red (Molecular Probes) ou dUTP-Cy3 (Amersham) ou dUTP-Cy5 (Amersham). Par contre, pour un marquage indirect on a utilisé du dUTP-16-biotinylé (Boehringer-Mannheim). Le marquage a été réalisé en tube Eppendorf (2 ml) sur la nuit à 15-16°C. Les nucléotides libres ont ensuite été éliminés par précipitation de la sonde à l'éthanol. Le culot d'ADN a été repris dans 500 µl de TE 10:1 (Tris-HCl 10 mM, EDTA 1 mM, pH8) afin que la sonde soit à une concentration d'environ 30 ng/µl.

### Composition du mélange des 23 sondes de peinture chromosomique

Chaque sonde spécifique d'un chromosome a été marquée individuellement avec les différents dUTP-modifiés (fluorescents ou non). En fonction de la richesse en bandes R et G de chacun des chromosomes et en fonction de leur taille, on a a priori établi ceux qui devaient ou non être composés de différents fluorochromes. Par exemple, des combinaisons de 3 ou 4 fluorochromes ont été de prégérence utilisées pour des chromosomes riches en bandes R (ex : chromosome 19), par contre pour des chromosomes pauvres en bandes R, seulement 1 à 2 fluorochromes ont été utilisés (ex : chromosome 8). Ce choix dépendait également, pour chaque fluorochrome, de la combinaison des filtres d'excitation et d'émission. En effet, lorsqu'une sonde a été marquée en proportion équivalente avec différents fluorochromes, les intensités de signal des différents fluorochromes ne sont pas forcément comparables. Cela dépend en effet de la qualité des filtres d'excitation et d'émission pour chaque fluorochrome, mais aussi de la quantité de fluorescence émise par le fluorochrome. Celle-ci dépend elle-même de l'intensité du flux lumineux à l'excitation et donc du pouvoir spectral de la source du lumière (Lampe Mercure HBO 100 W - OSRAM). Parmi tous ces paramètres, on a plutôt choisi d'optimiser le pouvoir de résolution des combinaisons de filtres, afin d'obtenir à l'émission le meilleur rapport signal/bruit de fond pour chaque fluorochrome. En effet, les différences d'intensité de fluorescence peuvent être compensées d'une part en augmentant ou diminuant les temps d'exposition au cours de l'acquisition de l'image par la caméra (Hamamatsu C4880), mais également en faisant varier les concentrations des sondes selon le marqueur fluorescent utilisé. Finalement, les concentrations des sondes ont été ajustées de sorte que pour un fluorochrome donné (et donc pour une combinaison de filtres données), touts les chromosomes marqués émettent une fluorescence d'intensité équivalente.

La composition des 23 sondes de peinture chromosomique a donc été définie expérimentalement et de façon précise après de nombreuses expériences de contrôle (tableau 2).

400 µg (environ 27 fois) d'ADN Cot1 (ADN compétiteur humain) ont été rajoutés à ces 1470 ng de mélange de sondes chromosomiques (50 sondes différentes). Le mélange d'ADN a ensuite été précipité à l'éthanol. Le culot a été repris dans 10 µl de mélange d'hybridation (50% formamide, 10% sulfate dextran, 2 X SSC (pH 7), 1 µg/µl d'ADN de sperme de hareng soniqué).

**Tableau 2**

| Fluor Chrom. | FITC | Cy3 | TR | Cy5 | Bio/Cy7 | Mélange 1470ng= |
|---|---|---|---|---|---|---|
| 1 | | 35 | | | | 35 |
| 2 | | | 40 | | | 40 |
| 3 | 30 | | | | 25 | 55 |
| 4 | | | | 50 | | 50 |
| 5 | 50 | | | | | 50 |
| 6 | | | | 40 | 30 | 70 |
| 7 | | | 30 | | 35 | 65 |
| 8 | 60 | | | 30 | | 90 |
| 9 | 30 | 20 | | | | 50 |
| 10 | | 30 | 25 | | 20 | 75 |
| 11 | | 30 | | 40 | | 70 |
| 12 | | | 35 | 50 | | 85 |
| 13 | 60 | | 45 | | | 105 |
| 14 | 25 | 20 | | | 25 | 70 |
| 15 | 50 | 15 | 15 | | | 80 |
| 16 | 30 | 20 | | 20 | | 70 |
| 17 | | 15 | | 25 | 20 | 60 |
| 18 | | | | | 40 | 40 |
| 19 | 25 | 10 | 20 | | 20 | 75 |
| 20 | | 20 | 20 | 30 | | 70 |
| 21 | | 30 | 30 | | | 60 |
| 22 | 10 | | 15 | 20 | | 45 |
| X | | 30 | | | 30 | 60 |

| | | | | | | |
|---|---|---|---|---|---|---|
| Nom complet des fluorochromes : FITC : Fluorescein isothiocyanate ; TR : Texas Red ; Cy3 : Cyanine 3 ; Cy5 : Cyanine 5 ; Bio : Biotine ; Cy7 : Cyanine 7 | | | | | | |

### Une alternative de préparation des sondes

De manière alternative, la préparation du mélange des sondes a été réalisée avant leur marquage par Nick translation pour chaque fluorochrome (Tableau 3). On a obtenu ainsi 5 mélanges de sondes qui ont été marqués par Nick translation à l'aide de nucléotides modifiés selon le protocole suivant.

1 à 2 µg du mélange de sondes ont été marqués par nick traslation dans un volume de 50 µl contenant : 20 µM de chacun des déoxynucléotides triphosphates (dATP, dGTP, dCTP), 10 µM de dTTP et 10 µM de dUTP modifié, Tris-HCl 50 µM, MgCl₂ 5 µM, β-mercaptoéthanol 1 µM et 20 U de mélange d'enzymes (Dnase/Polymérase : Boehringer-Mannheim). Pour marquer directement la sonde en fluorescence le nucléotide modifié a été du dUTP-12-FITC (Boehringer-Mannheim) ou dUTP-12-Texas red (Molecular Probes) ou dUTP-Cy3 (Amersham) ou dUTP-Cy5 (Amersham). Par contre, pour un marquage indirect on a utilisé du dUTP-16-biotinylé (Boehringer-Mannheim). Le marquage a été réalisé en tube Eppendorf (2 ml) sur la nuit à 15-16°C.

Après marquage, les 5 mélanges de sondes chromosomiques (14,75 µg) ont été précipités ensemble (à l'éthanol) en présence de 400 µg (environ 27 fois) d'ADN Cotl (ADN compétiteur humain). Le culot a été repris dans 10 µl de mélange d'hybridation (50% formamide, 10 % sulfate dextran, 2 X SSC (pH7), 1 µg/µl d'ADN de sperme de hareng soniqué).

Cette méthode alternative de marquage présente l'avantage d'alléger et de simplifier le protocole de marquage des sondes en réduisant le nombre de marquages par Nick translation à cinq au lieu de cinquante et en ne réalisant qu'un seul mélange de sondes au lieu de deux.

### 2. Hybridation in situ fluorescente

La procédure a été celle décrite par Cherif et al., 1990, avec quelques modifications.

### Préparation des chromosomes en métaphase

La préparation des chromosomes métaphasiques a été réalisée à partir d'une culture de lymphocytes circulants obtenus par ponction veineuse d'un sujet normal. Les lymphocytes stimulés par la phytohémagglutinine (PHA) (100 µl pour 8 ml de culture) ont été mis en culture pendant 72 heures à 37°C dans du milieu RPMI-1640. Les cellules ont ensuite été synchronisées en ajoutant du méthotrexate (10 µM) pendant 17 heures puis rincées et remises en culture en présence de 5-bromodéoxyuridine (BrdU) (0,1 mM) pendant 6 heures. Après action de la colchicine (1 mg/ml) pendant 15 min, l'éclatement des cellules a été obtenu par resuspension dans une solution hypotonique de KCl (75 mM). Les chromosomes ont été fixées dans un mélange méthanol/acide acétique (3 vol/l vol) et une à deux gouttes de suspension cellulaire ont été étalées sur chaque lame. Les lames séchées à température ambiante pendant 2 à 3 jours ont ensuite été stockées à -20°C pendant plusieurs mois.

**Tableau 3**

| Fluor. Chrom. | FITC | Cy3 | TR | Cy5 | Bio/Cy7 | Mélange |
|---|---|---|---|---|---|---|
| 1 | | 0,3 | | | | 0,3 |
| 2 | | | 0,4 | | | 0,4 |
| 3 | 0,3 | | | | 0,25 | 0,55 |
| 4 | | | | 0,5 | | 0,5 |
| 5 | 0,5 | | | | | 0,5 |
| 6 | | | | 0,4 | 0,3 | 0,7 |
| 7 | | | 0,3 | | 0,35 | 0,65 |
| 8 | 0,6 | | | 0,3 | | 0,9 |
| 9 | 0,3 | 0,2 | | | | 0,5 |
| 10 | | 0,3 | 0,25 | | 0,2 | 0,75 |
| 11 | | 0,3 | | 0,4 | | 0,7 |
| 12 | | | 0,35 | 0,5 | | 0,85 |
| 13 | 0,6 | | 0,45 | | | 1,05 |
| 14 | 0,25 | 0,2 | | | 0,25 | 0,7 |
| 15 | 0,5 | 0,15 | 0,15 | | | 0,8 |
| 16 | 0,3 | 0,2 | | 0,2 | | 0,7 |
| 17 | | 0,15 | | 0,25 | 0,2 | 0,6 |
| 18 | | | | | 0,4 | 0,4 |
| 19 | 0,25 | 0,1 | 0,2 | | 0,2 | 0,75 |
| 20 | | 0,2 | 0,2 | 0,3 | | 0,7 |
| 21 | | 0,3 | 0,3 | | | 0,6 |
| 22 | 0,1 | | 0,15 | 0,2 | | 0,45 |
| X | | 0,3 | | | 0,3 | 0,6 |
| Total (µg) | 3,7 | 2,7 | 2,75 | 3,15 | 2,45 | 14,75 |

| | | | | | | |
|---|---|---|---|---|---|---|
| Nom complet des fluorochromes : FITC : Fluorescein isothiocyanate ; TR : Texas Red ; Cy3 : Cyanine 3 ; Cy5 : Cyanine 5 ; Bio : Biotine ; Cy7 : Cyanine 7 | | | | | | |

### Préparation des lames

Les lames ont été traitées à l'ARNase (100 µg/ml) pendant 1 heure à 37°C puis rincées 3 fois (5 min. chaque) dans une solution de 2 X SSC, pH 7. Les lames ont ensuite été déshydratées par passages successifs dans une série de bains d'éthanol à concentration croissante (70%, 80%, 90% et 100% et 2 min. par bain), et séchées. La dénaturation de l'ADN chromosomique a été faite par immersion des lames dans un bain de formamide 70% / 2 X SSC (pH 7) à 70°C pendant 2 minutes. La dénaturation a été arrêtée en plongeant les lames (2 minutes) dans de l'éthanol 70% refroidi à - 20°C et maintenu dans un bac à glace. Puis les lames ont été déshydratées par passages successifs dans une série de bains d'éthanol à concentration croissante et séchées. Les lames ont ensuite été incubées pendant 8 à 10 min. à 37°C dans une solution (20 mM Tris-HCl, pH 7,4, 2 mM CaCl₂) contenant de la protéinase K (100 ng/ml) puis déshydratées dans une série de bains d'éthanol et séchées.

### Hybridation et détection du signal

Le mélange de sondes et d'ADN compétiteur (10 µl) a été dénaturé 10 min. à 70°C puis plongé dans de la glace et préhybridé pendant au moins 3 heures à 37°C. Ce mélange a ensuite été déposé sur la lame et recouvert d'une lamelle (18 X 22 mm). L'hybridation a eu lieu pendant 2-3 jours à 37°C en chambre humide.

Les lames ont ensuite été lavées dans une série de 3 bains de formamide 50%, 2 X SSC, pH 7 (3 min. chaque) à une température de 42-45°C, suivis de 5 lavages dans 2 X SSC, pH 7 (2 minutes chaque) et d'un lavage d'une minute dans une solution 1 X BN (0,1 M bicarbonate de sodium, 0,05% nonidet P-40). Les sondes biotinylées ont été détectées par adjonction d'avidine (Vector Laboratories Biosys) couplée à la cyanine7 (Amersham) (Avidine-Cy7) (5 µg/ml). Le couplage de l'avidine à la cyanine 7 a été réalisé avec un kit de couplage (Amersham). Afin de diminuer les problèmes de bruit de fond liés à des liaisons non spécifiques de l'avidine, les lames ont été préalablement incubées pendant 10 minutes dans une solution 1 X BN contenant 5% de lait en poudre écrémé. Les lames ont ensuite été incubées 1/2 heure à 37°C dans une solution contenant de l'avidine-Cy7 (5 µg/ml dans 1 X BN + 5% de lait en poudre) puis lavées successivement 3 fois (2 minutes) dans une solution 1 X BN à 45°C. Le signal fluorescent a été amplifié par adjonction d'une couche d'anticorps anti-avidine biotinylés (5 µg/ml) (Vector Laboratories, Biosys France), suivie d'une couche d'avidine-Cy7 (5 µg/ml) selon le protocole décrit par Pinkel et al., 1986. Pour chaque couche, les lames ont été incubées pendant 30 minutes à 37°C puis lavées 3 fois dans une solution 1 X BN. Les sondes marquées avec dUTP-FITC, dUTP-TR,dUTP-Cy3 et dUTP-Cy5 n'ont nécessité aucune étape de révélation supplémentaire.

L'observation des lames a été réalisée à l'aide d'un photomicroscope à épifluorescence (DMRX B, LEICA) équipé de la combinaison de filtres décrite précédemment. Deux roues indépendantes ont été utilisées pour porter 8 filtres chacune. La roue portant les filtres d'excitation a été insérée immédiatement après la lampe Hg et celle portant les filtres d'émission a été placée au dessus des objectifs et du filtre séparateur de faisceau. Avant l'acquisition des images, 20 µl d'une solution anti-fade (Johnson et al., 1981) ont été déposés sur chaque lame et recouverts d'une lamelle (solution anti-fade : 100 mg de PPD (p-phénylènediamine, Sigma) dans une solution composée de 10 ml de PBS et 90 ml de glycérol ; le pH de la solution a été ajusté à 8,0 avec du NaOH 0,1 M). La solution anti-fade permet d'éviter l'extinction rapide de la fluorescence émise par les différents fluorochromes lorsqu'ils sont soumis à une forte irradiation.

### 3. Exemple d'utilisation d'une combinaison de 6 fluorophores

La cyanine 7 étant un fluorochrome relativement instable, on a essayé de le remplacer par un autre fluorochrome, par exemple le Bodipy 630/650 (Molecular Probes). Couplé à un anticorps ou une molécule d'avidine, le Bodipy permet de révéler indirectement une sonde marquée à la biotine, à la digoxigénine ou au dinitrophénol (DNP). Dans ce cas, le choix des 5 fluorochromes pour la multifluorescence ne peut plus être le même car les spectres d'absorption et d'émission de la cyanine 5 et du Bodipy 630/650 sont trop proches pour qu'il y ait une bonne discrimination entre ces deux fluorochromes. Le choix sera le suivant :
- isothiocyanate fluoresceine (FITC)
- rouge Texas (TR)
- Cyanine 3 (Cy3)
- Bodipy 630/650
- Cyanine 5,5 (Cy5,5)

Ce choix a également l'avantage de permettre d'utiliser la cyanine 7 comme 6ème fluorochrome en cas de nécessité (par exemple pour des applications où l'on ne peut pas faire du marquage combinatoire de sonde, et où il serait avantageux d'avoir différentes sondes pouvant être discriminées avec un maximum de fluorochromes différents).

Dans ce cas, la Cy5,5 devra également être couplée à un anticorps ou une molécule d'avidine pour pouvoir révéler une sonde marquée à la biotine, à la digoxigénine ou au DNP.

Par exemple, pour le caryotypage en multifluorescence, les différents systèmes de marquage et de révélation des sondes seront :

| Marquage | Type de marquage | Révélation |
|---|---|---|
| isothiocyanate fluoresceine (FITC) | direct | non |
| rouge Texas (TR) | direct | non |
| Cyanine 3 (Cy3) | direct | non |
| digoxigénine (Dig) | indirect | anti dig-Bodipy 630/650 |
| biotine (Bio) | indirect | avidine-Cy5,5 |

Au cas où l'on souhaiterait utiliser 6 fluorochromes à la fois, le choix pourrait être :

| Marquage | Type de marquage | Révélation |
|---|---|---|
| isothiocyanate fluoresceine (FITC) | direct | non |
| rouge Texas (TR) | direct | non |
| Cyanine 3 (Cy3) | direct | non |
| dinitrophénol (DNP) | indirect | anti DNP-Bodipy 630/650 |
| digoxigénine (Dig) | indirect | anti dig-Cy5,5 |
| biotine (Bio) | indirect | avidine-Cy7 |

### TEXTE LIBRE DU LISTAGE DE SEQUENCE

primer PCR LINE
primer PCR Alu

### REFERENCES

Cherif D., Julier C., Delattre O., Derre J., Lathrop GM., Berger R., (1990). Simultaneous localization of cosmids and chromosome R-banding by fluorescence microscopy: Application to regional mapping of chromosome 11. Proc. Natl. Acad. Sci. USA 87, 6639.
Dutrillaux B., Lejeune J. (1971). Sur une nouvelle technique d'analyse du caryotype humain. C.R. Acad. Sci. 272, 2638.
Johnson G.D., De Nogueira C., Aranjo J.G.M. (1981). A simple method for reducing the fading of immunofluorescence during microscopy. J. Immunol. Methods 43, 349.
Korenberg J.R., Rikowski M.C. (1988). Human genome organization: Alu, Lines and the molecular structure of metaphase chromosome bands. Cell 53, 391.
Ledbetter S.A.,Garcia-Heras J., Ledbetter D.H. (1990). "PCR-karyotype" of human chromosomes in somatic cell hybrids. Genomics 8, 614.
Lengauer et al. (1990) Painting of human chromosomes with probes generated from hybrid cell lines by PCR with Alu and L1 primers. Human Genetics 86, 1-6.
Lichter P., Ledbetter S.A., Ledbetter D.H., Ward D.C. (1990). Fluorescence in situ hybridization with Alu and LI polymerase chain reaction probes for rapid characterization of human chromosomes in hybrid cell lines. Proc. Natl. Acad. Sci. USA 87, 6634.
Pinkel D., Straume T., Gray J.W. (1996). Cytogenetic analysis using quantitative, high sensitivity fluorescence hybridization. Proc. Natl. Acad. Sci. USA 83, 2934.
Richard P Haugland, 1996, Molecular Probes, MTZ Spence Ed. « Handbook of fluorescent probes and research chemicals »
Romana S.P., Tachdjian G., Druart L., Cohen D., Berger R., Cherif D. (1993). A simple method for prenatal diagnosis of trisomy 21 on uncultured amniocytes. Eur. J. Hum. Genet. 1, 245.
SabileA., Poras I., Cherif D., Goodfellow P., Avner P. (1997). Isolation of monochromosomal hybrid for mouse chromosomes 3,6, 10, 12, 14 and 18. Mammalian Genome 8, 81.
Schröck E., du Manoir S., Veldman T., Schoell B., Wienberg J., Ferguson-Smith M.A., Ning Y., Ledbetter D.H., Bar-Am I., Soenksen D., Garini Y., Ried T. (1996). Multicolor spectral karyotyping of human chromosomes. Science 273, 494-497.
Speicher M.R., Ballard S.G., Ward DC. (1996). Karyotyping human chromosomes by combinatorial multi-fluor FISH. Nature Genetics 12, 368-375.
Summer A.T., Evans H.J., Buckland R.A. (1971). A new technique for distinguishing between human chromosomes. Nature (New Biol) 232, 31.

## Revendications

1. Mélange de Sondes destinées au marquage d'un chromosome, constitué par un mélange de deux produits d'amplification IRS-PCR à partir dudit chromosome composé d'une part d'un:
- produit d'amplification PCR obtenu par l'utilisation de l'amorce spécifique des séquences d'ADN Alu ou de l'amorce spécifique des séquences d'ADN LINE,
et d'autre part d'un:
- produit d'amplification PCR obtenu par l'utilisation de l'amorce spécifique des séquences d'ADN Alu et de l'amorce spécifique des séquences d'ADN LINE.

2. Mélange de Sondes selon la revendication 1, constitué par un mélange de deux produits d'amplification IRS-PCR composé d'une part d'un:
- produit d'amplification PCR obtenu par l'utilisation de l'amorce spécifique des séquences d'ADN Alu,
et d'autre part d'un:
- produit d'amplification PCR obtenu par l'utilisation de l'amorce spécifique des séquences d'ADN Alu et de l'amorce spécifique des séquences d'ADN LINE.

3. Mélange de Sondes selon la revendication 1, constitué par un mélange de deux produits d'amplification IRS-PCR composé d'une part d'un:
- produit d'amplification PCR obtenu par l'utilisation de l'amorce spécifique des séquences d'ADN LINE,
et d'autre part d'un:
- produit d'amplification PCR obtenu par l'utilisation de l'amorce spécifique des séquences d'ADN Alu et de l'amorce spécifique des séquences d'ADN LINE.

4. Mélange de Sondes selon l'une des revendications 1 à 3, **caractérisé en ce que** les segments d'ADN amplifiés par IRS-PCR ont pour source des hybrides somatiques rongeur/homme monochromosomiques.

5. Mélange de Sondes selon l'une des revendications 1 à 4, **caractérisé en ce que** lesdites sondes sont spécifiques de chromosome humain.

6. Mélange de Sondes selon l'une des revendications 1 à 5, **caractérisé en ce que** lesdites amorces spécifiques utilisées comprennent ou sont les amorces de séquences :
- SEQ ID N° 1 pour l'amorce spécifique des séquences d'ADN Alu,
- SEQ ID N°^{s} 2 et 3 pour l'amorce spécifique des séquences d'ADN LINE.

7. Mélange de Sondes selon l'une des revendications 1 à 6, **caractérisé en ce que** les segments d'ADN sont marqués directement ou indirectement par les techniques de fluorescence.

8. Mélange de Sondes ADN selon la revendication 7 **caractérisées en ce que** les segments d'ADN sont marqués par au moins un fluorophore choisi parmi l'isothiocyanate de fluorescine (FITC), le rouge Texas (TR), la cyanine 3 (Cy3), la cyanine 5 (Cy5), la cyanine 5,5 (Cy5,5), la cyanine 7 (Cy7), le Bodipy 630/650.

9. Ensemble de mélanges de sondes destinées au marquage des chromosomes humains, **caractérisé en ce qu'**il contient des mélanges de sondes selon l'une des revendications 1 à 8 pour chacun des chromosomes humains.

10. Procédé de production de mélange de sondes destinées au marquage de chromosomes humains, **caractérisé en ce que** ledit procédé comprend les étapes suivantes :
- effectuer deux amplifications IRS-PCR à partir desdits chromosomes en utilisant d'une part des amorces PCR spécifiques des séquences d'ADN Alu et LINE, et d'autre part des amorces PCR spécifiques des séquences d'ADN Alu ou des amorces PCR spécifiques des séquences d'ADN LINE; et
- mélanger les deux produits d'amplification.

11. Procédé selon la revendication 10, **caractérisée en ce que** les deux amplifications IRS-PCR utilisent d'une part des amorces PCR spécifiques des séquences d'ADN Alu et LINE, et d'autre part des amorces PCR spécifiques des séquences d'ADN Alu.

12. Procédé selon la revendication 10, **caractérisée en ce que** les deux amplifications IRS-PCR utilisent d'une part des amorces PCR spécifiques des séquences d'ADN Alu et LINE, et d'autre part des amorces PCR spécifiques des séquences d'ADN LINE.

13. Utilisation d'un mélange de sondes selon l'une des revendications 1 à 8 ou d'un ensemble de mélanges de sondes selon la revendication 9 pour la peinture et le marquage en bandes cytogénétiques d'un chromosome.

14. Procédé de FISH multicouleur, **caractérisé en ce qu'**on utilise pour sa mise en oeuvre des mélanges de sondes selon l'une des revendications 1 à 8 ou un ensemble de mélanges de sondes selon la revendication 9.

15. Procédé selon la revendication 14, **caractérisé en ce que** les sondes d'ADN sont marquées par des fluorophores et **en ce que** chaque fluorophore de longueur d'onde d'absorption et d'émission spécifique est associé à un couple de filtres optiques, un pour l'absorption et un pour l'émission, ledit procédé utilisant des fluorophores et des couples de filtres choisis parmi le groupe suivant :
a) le fluorophore FITC ayant une longueur d'onde maximale d'absorption de 494 nm et une longueur d'onde maximale d'émission de 517 nm associé à un filtre à l'excitation de type 490DF30 (Omega Optical) et à un filtre à l'émission de type 530DF30 (Omega Optical),
b) le fluorophore Cy3 ayant une longueur d'onde maximale d'absorption de 554 nm et une longueur d'onde maximale d'émission de 568 nm associé à un filtre à l'excitation de type 546DF10 (Omega Optical) et à un filtre à l'émission de type 570DF10 (Omega Optical),
c) le fluorophore TR ayant une longueur d'onde maximale d'absorption de 593 nm et une longueur d'onde maximale d'émission de 613 nm associé à un filtre à l'excitation de type 590DF10 (Omega Optical) et à un filtre à l'émission de type 615DF10 (Omega Optical),
d) le fluorophore Cy5 ayant une longueur d'onde maximale d'absorption de 652 nm et une longueur d'onde maximale d'émission de 670 nm associé à un filtre à l'excitation de type 650DF20 (Omega Optical) et à un filtre à rémission de type 670DF10 (Omega Optical),
e) le fluorophore Cy7 ayant une longueur d'onde maximale d'absorption de 743 nm et une longueur d'onde maximale d'émission de 767 nm associé à un filtre à l'excitation de type 740DF25 (Omega Optical) et à un filtre à l'émission de type 780EFLP (Omega Optical),
f) le fluorophore Cy5,5 ayant une longueur d'onde maximale d'absorption de 675 nm et une longueur d'onde maximale d'émission de 694 nm associé à un filtre à l'excitation de type 680DF20 (Omega Optical) et à un filtre à l'émission de type 700EFLP (Omega Optical),
g) le fluorophore Bodipy 630/650 ayant une longueur d'onde maximale d'absorption de 632 nm et une longueur d'onde maximale d'émission de 658 nm associé à un filtre à l'excitation de type 630DF20 (Omega Optical) et à un filtre à l'émission de type 650DF10 (Omega Optical).

16. Procédé selon la revendication 15, **caractérisé en ce que** les filtres optiques présentent les qualités suivantes :
- ils sont de type 6 cavités,
- ils ont une ADI de 0°;
- ils ont une tolérance λ₀ ± 20% de FWHM,
- ils ont une tolérance sur FWHM de ± 20% de FWHM,
- ils ont une réjection hors bande passante OD5 de UV à 1200 nm
- ils ont une courbe de transmission T ≥ 50% à λ₀.

17. Procédé selon la revendication 16, **caractérisé en ce que** les filtres optiques présentent en outre les caractéristiques suivantes :
- ils ont un diamètre utile centré supérieur à 21 mm,
- ils ont une épaisseur ≤ 7 mm.

18. Procédé selon l'une des revendications 14 à 17, **caractérisé en ce que** lesdites sondes sont utilisées pour l'étude des caryotypes et des caryotypes de réarrangements chromosomiques.

19. Kit de diagnostic **caractérisé en ce qu'**il comprend au moins des mélanges de sondes ADN telles que décrites dans les revendications 1 à 8 ou un ensemble de mélanges de sondes selon la revendication 9.

20. Kit selon la revendication 19, **caractérisé en ce que** les sondes d'ADN sont marquées par des fluorophores et **en ce que** chaque fluorophore de longueur d'onde d'absorption et d'émission spécifique est associé à un couple de filtres optiques, un pour l'absorption et un pour l'émission, ledit kit utilisant des fluorophores et des couples de filtres choisis parmi le groupe suivant :
a) le fluorophore FTTC ayant une longueur d'onde maximale d'absorption de 494 nm et une longueur d'onde maximale d'émission de 517 nm associé à un filtre à l'excitation de type 490DF30 (Omega Optical) et à un filtre à l'émission de type 530DF30 (Omega Optical),
b) le fluorophore Cy3 ayant une longueur d'onde maximale d'absorption de 554 nm et une longueur d'onde maximale d'émission de 568 nm associé à un filtre à l'excitation de type 546DF10 (Omega Optical) et à un filtre à l'émission de type 570DF10 (Omega Optical),
c) le fluorophore TR ayant une longueur d'onde maximale d'absorption de 593 nm et une longueur d'onde maximale d'émission de 613 nm associé à un filtre à l'excitation de type 590DF10 (Omega Optical) et à un filtre à l'émission de type 615DF10 (Omega Optical),
d) le fluorophore Cy5 ayant une longueur d'onde maximale d'absorption de 652 nm et une longueur d'onde maximale démission de 670 nm associé à un filtre à l'excitation de type 650DF20 (Omega Optical) et à un filtre à l'émission de type 670DF10 (Omega Optical),
e) le fluorophore Cy7 ayant une longueur d'onde maximale d'absorption de 743 nm et une longueur d'onde maximale d'émission de 767 nm associé à un filtre à l'excitation de type 740DF25 (Omega Optical) et à un filtre à l'émission de type 780EFLP (Omega Optical),
f) le fluorophore Cy5,5 ayant une longueur d'onde maximale d'absorption de 675 nm et une longueur d'onde maximale d'émission de 694 nm associé à un filtre à l'excitation de type 680DF20 (Omega Optical) et à un filtre à l'émission de type 700EFLP (Omega Optical),
g) le fluorophore Bodipy 630/650 ayant une longueur d'onde maximale d'absorption de 632 nm et une longueur d'onde maximale d'émission de 658 nm associé à un filtre à l'excitation de type 630DF20 (Omega Optical) et à un filtre à l'émission de type 650DF10 (Omega Optical).

## Claims

1. Mixture of probes intended for the labelling of a chromosome, consisting of a mixture of two IRS-PCR amplification products from said chromosome, composed, in one part, of a:
- PCR amplification product obtained using the primer specific for Alu DNA sequences or the primer specific for LINE DNA sequences, and, in the other part, of a:
- PCR amplification product obtained using the primer specific for Alu DNA sequences and the primer specific for LINE DNA sequences.

2. Mixture of probes according to claim 1, consisting of a mixture of two IRS-PCR amplification products composed, in one part, of a:
- PCR amplification product obtained using the primer specific for Alu DNA sequences, and, in the other part, of a:
- PCR amplification product obtained using the primer specific for Alu DNA sequences and the primer specific for LINE DNA sequences.

3. Mixture of probes according to claim 1, consisting of a mixture of two IRS-PCR amplification products composed, in one part, of a:
- PCR amplification product obtained using the primer specific for LINE DNA sequences, and, in the other part, of a:
- PCR amplification product obtained using the primer specific for Alu DNA sequences and the primer specific for LINE DNA sequences.

4. Mixture of probes according to any one of claims 1 to 3, **characterised in that** the DNA segments amplified by IRS-PCR have as their source monochromosomal rodent/human somatic hybrids

5. Mixture of probes according to any one of claims 1 to 4, **characterised in that** said probes are specific for a human chromosome.

6. Mixture of probes according to any one of claims 1 to 5, **characterised in that** said specific primers used comprise or are the primers of sequences:
- SEQ ID No. 1 for the primer specific for Alu DNA sequences,
- SEQ ID Nos. 2 and 3 for the primer specific for LINE DNA sequences.

7. Mixture of probes according to any one of claims 1 to 6, **characterised in that** the DNA segments are labelled directly or indirectly by fluorescence techniques.

8. Mixture of DNA probes according to claim 7, **characterised in that** the DNA segments are labelled with at least one fluorophore chosen from fluorescein isothiocyanate (FITC), Texas Red (TR), cyanine 3 (Cy3), cyanine 5 (Cy5), cyanine 5.5 (Cy5.5), cyanine 7 (Cy7), Bodipy 630/650.

9. Set of mixtures of probes intended for the labelling of human chromosomes, **characterised in that** it contains mixtures of probes according to any one of claims 1 to 8 for each of the human chromosomes.

10. Method of producing a mixture of probes intended for labelling human chromosomes, **characterised in that** said method comprises the following steps:
- carrying out two IRS-PCR amplifications from said chromosomes using, in one part, PCR primers specific for Alu and LINE DNA sequences and, in the other part, PCR primers specific for Alu DNA sequences or PCR primers specific for LINE DNA sequences; and
- mixing the two amplification products.

11. Method according to claim 10, **characterised in that** the two IRS-PCR amplifications use, in one part, PCR primers specific for Alu and LINE DNA sequences and, in the other part, PCR primers specific for Alu DNA sequences.

12. Method according to claim 10, **characterised in that** the two IRS-PCR amplifications use, in one part, PCR primers specific for Alu and LINE DNA sequences and, in other part, PCR primers specific for LINE DNA sequences.

13. Use of a mixture of probes according to any one of claims 1 to 8 or of a set of mixtures of probes according to claim 9 for the painting and the labelling in cytogenetic bands of a chromosome.

14. Multicolour FISH method, **characterised in that** its implementation involves the use of mixtures of probes according to any one of claims 1 to 8 or of a set of mixtures of probes according to claim 9.

15. Method according to claim 14, **characterised in that** the DNA probes are labelled with fluorophores and **in that** each fluorophore having a specific absorption and emission wavelength is associated with a pair of optical filters, one for absorption and one for emission, said method using fluorophores and pairs of filters chosen from the following group:
a) the fluorophore FITC having a maximum absorption wavelength of 494 nm and a maximum emission wavelength of 517 nm associated with an excitation filter of the 490DF30 type (Omega Optical) and with an emission filter of the 530DF30 type (Omega Optical),
b) the fluorophore Cy3 having a maximum absorption wavelength of 554 nm and a maximum emission wavelength of 568 nm associated with an excitation filter of the 546DF10 type (Omega Optical) and with an emission filter of the 570DF10 type (Omega Optical),
c) the fluorophore TR having a maximum absorption wavelength of 593 nm and a maximum emission wavelength of 613 nm associated with an excitation filter of the 590DF10 type (Omega Optical) and with an emission filter of the 615DF10 type (Omega Optical),
d) the fluorophore Cy5 having a maximum absorption wavelength of 652 nm and a maximum emission wavelength of 670 nm associated with an excitation filter of the 650DF20 type (Omega Optical) and with an emission filter of the 670DF10 type (Omega Optical),
e) the fluorophore Cy7 having a maximum absorption wavelength of 743 nm and a maximum emission wavelength of 767 nm associated with an excitation filter of the 740DF25 type (Omega Optical) and with an emission filter of the 780EFLP type (Omega Optical),
f) the fluorophore Cy5.5 having a maximum absorption wavelength of 675 nm and a maximum emission wavelength of 694 nm associated with an excitation filter of the 680DF20 type (Omega Optical) and with an emission filter of the 700EFLP type (Omega Optical),
g) the fluorophore Bodipy 630/650 having a maximum absorption wavelength of 632 nm and a maximum emission wavelength of 658 nm associated with an excitation filter of the 630DF20 type (Omega Optical) and with an emission filter of the 650DF10 type (Omega Optical).

16. Method according to claim 15, **characterised in that** the optical filters have the following qualities:
- they are of the 6-cavity type,
- they have an ADI of 0°;
- they have a tolerance λ₀ ± 20% of FWHM,
- they have a tolerance on FWHM of ± 20% of FWHM,
- they have an OD5 out-of-passband rejection for UV at 1,200 nm,
- they have a transmission curve T ≥ 50% at λ₀.

17. Method according to claim 16, **characterised in that** the optical filters also have the following characteristics:
- they have a centred useful diameter greater than 21 mm,
- they have a thickness ≤ 7 mm.

18. Method according to any one of claims 14 to 17, **characterised in that** said probes are used for studying karyotypes and the karyotypes of chromosomal rearrangements.

19. Diagnostic kit, **characterised in that** it comprises at least the mixtures of DNA probes as described in claims 1 to 8 or a set of mixtures of probes according to claim 9.

20. Kit according to claim 19, **characterised in that** the DNA probes are labelled with fluorophores and **in that** each fluorophore having a specific absorption and emission wavelength is associated with a pair of optical filters, one for absorption and one for emission, said kit using fluorophores and pairs of filters chosen from the following group:
a) the fluorophore FITC having a maximum absorption wavelength of 494 nm and a maximum emission wavelength of 517 nm associated with an excitation filter of the 490DF30 type (Omega Optical) and with an emission filter of the 530DF30 type (Omega Optical),
b) the fluorophore Cy3 having a maximum absorption wavelength of 554 nm and a maximum emission wavelength of 568 nm associated with an excitation filter of the 546DF10 type (Omega Optical) and with an emission filter of the 570DF10 type (Omega Optical),
c) the fluorophore TR having a maximum absorption wavelength of 593 nm and a maximum emission wavelength of 613 nm associated with an excitation filter of the 590DF10 type (Omega Optical) and with an emission filter of the 615DF10 type (Omega Optical),
d) the fluorophore Cy5 having a maximum absorption wavelength of 652 nm and a maximum emission wavelength of 670 nm associated with an excitation filter of the 650DF20 type (Omega Optical) and with an emission filter of the 670DF10 type (Omega Optical),
e) the fluorophore Cy7 having a maximum absorption wavelength of 743 nm and a maximum emission wavelength of 767 nm associated with an excitation filter of the 740DF25 type (Omega Optical) and with an emission filter of the 780EFLP type (Omega Optical),
f) the fluorophore Cy5.5 having a maximum absorption wavelength of 675 nm and a maximum emission wavelength of 694 nm associated with an excitation filter of the 680DF20 type (Omega Optical) and with an emission filter of the 700EFLP type (Omega Optical),
g) the fluorophore Bodipy 630/650 having a maximum absorption wavelength of 632 nm and a maximum emission wavelength of 658 nm associated with an excitation filter of the 630DF20 type (Omega Optical) and with an emission filter of the 650DF10 type (Omega Optical).

## Patentansprüche

1. Gemisch zum Markieren eines Chromosoms bestimmter Sonden, das aus einem Gemisch zweier IRS-PCR-Amplifizierungsprodukte aus dem Chromosom gebildet wird und sich zum einen aus
einem durch Verwenden eines für Alu-DNA-Sequenzen spezifischen Primers oder für LINE-DNA-Sequenzen spezifischen Primers erhaltenen PCR-Amplifizierungsprodukt und zum anderen aus
einem durch Verwenden eines für Alu-DNA-Sequenzen spezifischen Primers und für LINE-DNA-Sequenzen spezifischen Primers erhaltenen PCR-Amplifizierungsprodukt zusammensetzt.

2. Sondengemisch gemäß Anspruch 1, das aus einem Gemisch zweier IRS-PCR-Amplifizierungsprodukte gebildet wird und sich zum einen aus
einem durch Verwenden eines für Alu-DNA-Sequenzen spezifischen Primers erhaltenen PCR-Amplifizierungsprodukt
und zum anderen aus
einem durch Verwenden eines für Alu-DNA-Sequenzen spezifischen Primers und für LINE-DNA-Sequenzen spezifischen Primers erhaltenen PCR-Amplifizierungsprodukt zusammensetzt.

3. Sondengemisch gemäß Anspruch 1, das aus einem Gemisch zweier IRS-PCR-Amplifizierungsprodukte gebildet wird und sich zum einen aus
einem durch Verwenden eines für LINE-DNA-Sequenzen spezifischen Primers erhaltenen PCR-Amplifizierungsprodukt
und andererseits aus
einem durch Verwenden eines für Alu-DNA-Sequenzen spezifischen Primers und für LINE-DNA-Sequenzen spezifischen Primers erhaltenen PCR-Amplifizierungsprodukt zusammensetzt.

4. Sondengemisch gemäß einem der Ansprüche 1 bis 3, **dadurch gekennzeichnet, daß** die durch IRS-PCR amplifizierten DNA-Segmente somatische monochromosomale Nager/Human-Hybride als Ursprung aufweisen.

5. Sondengemisch gemäß einem der Ansprüche 1 bis 4, **dadurch gekennzeichnet, daß** die Sonden für ein humanes Chromosom spezifisch sind.

6. Sondengemisch gemäß einem der Ansprüche 1 bis 5, **dadurch gekennzeichnet, daß** die verwendeten spezifischen Primer die folgenden Primersequenzen umfassen oder sind:
- SEQ ID Nr. 1 für den auf Alu-DNA-Sequenzen spezifischen Primer,
- SEQ ID Nr. 2 und 3 für den auf LINE-DNA-Sequenzen spezifischen Primer.

7. Sondengemisch gemäß einem der Ansprüche 1 bis 6, **dadurch gekennzeichnet, daß** die DNA-Segmente durch Fluoreszenztechniken direkt oder indirekt markiert sind.

8. DNA-Sondengemisch gemäß Anspruch 7, **dadurch gekennzeichnet, daß** die DNA-Segmente durch wenigstens einen Fluorophor markiert sind, der aus Fluoresceinisothiocyanat (FITC), Texasrot (TR), Cyanin 3 (Cy3), Cyanin 5 (Cy5), Cyanin 5,5 (Cy5,5), Cyanin 7 (Cy7) und Bodipy 630/650 ausgewählt ist.

9. Menge an Sondengemischen zur Markierung humaner Chromosomen, **dadurch gekennzeichnet, daß** sie Sondengemische gemäß einem der Ansprüche 1 bis 8 für jedes humane Chromosom enthält.

10. Verfahren zur Herstellung von Sondengemischen zur Markierung humaner Chromosomen, **dadurch gekennzeichnet, daß** das Verfahren die folgenden Schritte umfaßt:
- Ausführen zweier IRS-PCR-Amplifizierungen aus den Chromosomen unter Verwenden zum einen für Alu- und LINE-DNA-Sequenzen spezifischer PCR-Primer und zum anderen für Alu-DNA-Sequenzen spezifischer PCR-Primer oder für LINE-DNA-Sequenzen spezifischer PCR-Primer und
- Mischen der beiden Amplifizierungsprodukte.

11. Verfahren gemäß Anspruch 10, **dadurch gekennzeichnet, daß** die beiden IRS-PCR-Amplifizierungen zum einen für Alu- und LINE-DNA-Sequenzen spezifische PCR-Primer und zum anderen für Alu-DNA-Sequenzen spezifische PCR-Primer verwenden.

12. Verfahren gemäß Anspruch 10, **dadurch gekennzeichnet, daß** die beiden IRS-PCR-Amplifizierungen zum einen für Alu- und LINE-DNA-Sequenzen spezifische PCR-Primer und zum anderen für LINE-DNA-Sequenzen spezifische PCR-Primer verwenden.

13. Verwendung eines Sondengemischs gemäß einem der Ansprüche 1 bis 8 oder einer Menge von Sondengemischen gemäß Anspruch 9 zum Anfärben und zum Markieren zytogenetischer Banden eines Chromosoms.

14. Mehrfarben-FISH-Verfahren, **dadurch gekennzeichnet, daß** zu seiner Ausführung Sondengemische gemäß einem der Ansprüche 1 bis 8 oder eine Menge von Sondengemischen gemäß Anspruch 9 verwendet werden.

15. Verfahren gemäß Anspruch 14, **dadurch gekennzeichnet, daß** die DNA-Sonden durch Fluorophore markiert sind und **dadurch**, daß jeder Fluorophor mit einer spezifischen Absorptions- und Emissionswellenlänge mit einem Paar optischer Filter, einem für die Absorption und einem für die Emission, verbunden ist, wobei das Verfahren Fluorophore und Filterpaare verwendet, die aus der folgenden Gruppe ausgewählt sind:
a) dem Fluorophor FITC mit einem Maximum der Absorptionswellenlänge von 494 nm und einem Maximum der Emissionswellenlänge von 517 nm kombiniert mit einem Anregungsfilter des Typs 490DF30 (Omega Optical) und mit einem Emissionsfilter des Typs 530DF30 (Omega Optical),
b) dem Fluorophor Cy3 mit einem Maximum der Absorptionswellenlänge von 554 nm und einem Maximum der Emissionswellenlänge von 568 nm kombiniert mit einem Anregungsfilter des Typs 546DF10 (Omega Optical) und mit einem Emissionsfilter des Typs 570DF10 (Omega Optical),
c) dem Fluorophor TR mit einem Maximum der Absorptionswellenlänge von 593 nm und einem Maximum der Emissionswellenlänge von 613 nm kombiniert mit einem Anregungsfilter des Typs 590DF10 (Omega Optical) und mit einem Emissionsfilter des Typs 615DF10 (Omega Optical),
d) dem Fluorophor Cy5 mit einem Maximum der Absorptionswellenlänge von 652 nm und einem Maximum der Emissionswellenlänge von 670 nm kombiniert mit einem Anregungsfilter des Typs 650DF20 (Omega Optical) und mit einem Emissionsfilter des Typs 670DF10 (Omega Optical),
e) dem Fluorophor Cy7 mit einem Maximum der Absorptionswellenlänge von 743 nm und einem Maximum der Emissionswellenlänge von 767 nm kombiniert mit einem Anregungsfilter des Typs 740DF25 (Omega Optical) und mit einem Emissionsfilter des Typs 780EFLP (Omega Optical),
f) dem Fluorophor Cy5,5 mit einem Maximum der Absorptionswellenlänge von 675 nm und einem Maximum der Emissionswellenlänge von 694 nm kombiniert mit einem Anregungsfilter des Typs 680DF20 (Omega Optical) und mit einem Emissionsfilter des Typs 700EFLP (Omega Optical),
g) dem Fluorophor Bodipy 630/650 mit einem Maximum der Absorptionswellenlänge von 632 nm und einem Maximum der Emissionswellenlänge von 658 nm kombiniert mit einem Anregungsfilter des Typs 630DF20 (Omega Optical) und mit einem Emissionsfilter des Typs 650DF10 (Omega Optical).

16. Verfahren gemäß Anspruch 15, **dadurch gekennzeichnet, daß** die optischen Filter die folgenden Eigenschaften aufweisen:
- sie sind vom 6-Cavity-Typ,
- sie weisen eine ADI von 0 ° auf,
- sie weisen eine Toleranz von λ₀ ± 20 % der FWHM auf,
- sie weisen eine Toleranz bei der FWHM von ± 20 % der FWHM auf,
- sie weisen eine Unterdrückung außerhalb des Durchlaßbereichs von OD5 für UV bei 1200 nm auf,
- sie weisen eine Durchlässigkeitskurve T ≥ 50 % bei λ₀ auf.

17. Verfahren gemäß Anspruch 16, **dadurch gekennzeichnet, daß** die optischen Filter außerdem die folgenden Merkmale aufweisen:
- sie weisen einen zentrierten nutzbaren Durchmesser größer als 21 mm auf,
- sie weisen eine Dicke ≤ 7 mm auf.

18. Verfahren gemäß einem der Ansprüche 14 bis 17, **dadurch gekennzeichnet, daß** die Sonden zur Untersuchung von Karyotypen und Karyotypen von Chromosomenumlagerungen verwendet werden.

19. Diagnosekit, **dadurch gekennzeichnet, daß** er wenigstens wie in den Ansprüchen 1 bis 8 beschriebene Gemische von DNA-Sonden oder eine Menge von Sondengemischen gemäß Anspruch 9 umfaßt.

20. Kit gemäß Anspruch 19, **dadurch gekennzeichnet, daß** die DNA-Sonden mit Fluorophoren markiert sind, und **dadurch**, daß jeder Fluorophor mit einer spezifischen Absorptions- und Emissionswellenlänge mit einem Paar optischer Filter, einem zur Absorption und einem zur Emission, verbinden ist, wobei der Kit Fluorophore und Filterpaare verwendet, die aus der folgenden Gruppe ausgewählt sind:
a) dem Fluorophor FITC mit einem Maximum der Absorptionswellenlänge von 494 nm und einem Maximum der Emissionswellenlänge von 517 nm kombiniert mit einem Anregungsfilter des Typs 490DF30 (Omega Optical) und mit einem Emissionsfilter des Typs 530DF30 (Omega Optical),
b) dem Fluorophor Cy3 mit einem Maximum der Absorptionswellenlänge von 554 nm und einem Maximum der Emissionswellenlänge von 568 nm kombiniert mit einem Anregungsfilter des Typs 546DF10 (Omega Optical) und mit einem Emissionsfilter des Typs 570DF10 (Omega Optical),
c) dem Fluorophor TR mit einem Maximum der Absorptionswellenlänge von 593 nm und einem Maximum der Emissionswellenlänge von 613 nm kombiniert mit einem Anregungsfilter des Typs 590DF10 (Omega Optical) und mit einem Emissionsfilter des Typs 615DF10 (Omega Optical),
d) dem Fluorophor Cy5 mit einem Maximum der Absorptionswellenlänge von 652 nm und einem Maximum der Emissionswellenlänge von 670 nm kombiniert mit einem Anregungsfilter des Typs 650DF20 (Omega Optical) und mit einem Emissionsfilter des Typs 670DF10 (Omega Optical),
e) dem Fluorophor Cy7 mit einem Maximum der Absorptionswellenlänge von 743 nm und einem Maximum der Emissionswellenlänge von 767 nm kombiniert mit einem Anregungsfilter des Typs 740DF25 (Omega Optical) und mit einem Emissionsfilter des Typs 780EFLP (Omega Optical),
f) dem Fluorophor Cy5,5 mit einem Maximum der Absorptionswellenlänge von 675 nm und einem Maximum der Emissionswellenlänge von 694 nm kombiniert mit einem Anregungsfilter des Typs 680DF20 (Omega Optical) und mit einem Emissionsfilter des Typs 700EFLP (Omega Optical),
g) dem Fluorophor Bodipy 630/650 mit einem Maximum der Absorptionswellenlänge von 632 nm und einem Maximum der Emissionswellenlänge von 658 nm kombiniert mit einem Anregungsfilter des Typs 630DF20 (Omega Optical) und mit einem Emissionsfilter des Typs 650DF10 (Omega Optical).
